(19) **Europäisches Patentamt / European Patent Office / Office européen des brevets**

(11) **EP 1 859 826 A1**

(12) **EUROPEAN PATENT APPLICATION**
published in accordance with Art. 158(3) EPC

(43) Date of publication:
**28.11.2007 Bulletin 2007/48**

(51) Int Cl.:
*A61M 1/36* (2006.01)   *A61K 49/04* (2006.01)

(21) Application number: **06714219.0**

(22) Date of filing: **21.02.2006**

(86) International application number:
**PCT/JP2006/303079**

(87) International publication number:
**WO 2006/090706 (31.08.2006 Gazette 2006/35)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.02.2005 JP 2005045726**
**28.09.2005 JP 2005282076**
**13.10.2005 JP 2005298986**

(71) Applicant: **Kaneka Corporation**
**Kita-ku**
**Osaka-shi, Osaka 530-8288 (JP)**

(72) Inventors:
• **MICHISHITA, Ichiro**
**Kanagawa, 2470007 (JP)**

• **FUJII, Zenzo**
**Yamaguchi, 7550076 (JP)**
• **HANITA, Sakiko**
**inishi, Settsu-shi, Osaka, 5660072 (JP)**
• **OGINO, Eiji**
**inishi, Settsu-shi, Osaka, 5660072 (JP)**
• **NISHIDE, Takuji**
**inishi, Settsu-shi, Osaka, 5660072 (JP)**

(74) Representative: **Gille Hrabal Struck Neidlein Prop Roos**
**Brucknerstrasse 20**
**40593 Düsseldorf (DE)**

(54) **CONTRAST AGENT-REMOVAL SYSTEM AND METHOD OF ACTIVATING THE CONTRAST AGENT-REMOVAL SYSTEM**

(57) The present invention aims to provide a contrast agent-removal system and a method of operating the system for efficiently removing the contrast agent which is cause of the renal function impairment. The system of the present invention includes a blood suction catheter which can be placed into the coronary sinus, a column for removing a contrast agent from the drawn blood, a blood drawing circuit for directing the blood into the column, a blood pump for controlling an amount of the drawn blood, a blood return circuit for returning the blood from which the contrast agent is removed to a patient's body and a device for measuring a blood suction pressure. In the method of operating the system, the blood is drawn with the blood suction catheter placed into the coronary sinus and the drawing blood is contacted with an adsorbent for adsorbing the contrast agent.

FIG. 1

EP 1 859 826 A1

**Description**

TECHNICAL FIELD

The present invention relates to a contrast agent-removal system for removing a contrast agent injected into a coronary artery and a method of activating the contrast agent-removal system.

BACKGROUND ART

Percutaneous Coronary Intervention (PCI) is generally carried out as a safe and an effective therapeutic method of dilating an arctation region or an occlusion region in blood vessel and ameliorating blood flow in a peripheral side of the blood vessel in case that the arctation or the occlusion is generated in a vessel such as blood vessel and the blood vessel is occluded by thrombus. Moreover, the therapeutic method has been carried out for difficult lesions such as left main trunk lesion of coronary artery (LMT) or chronic total occlusion (CTO) since PCI has been remarkably developed recently. Furthermore, a therapeutic method of placing a metallic stent or a drug eluting stent (DES) is also going to be established to retain the deflating of the arctation or the deflation of the occlusion region and prevent restenosis.

Although the contrast agent is essential for the PCI and the preoperative examinations, a side effect caused by the contrast agent to the patients with the renal function impairment is a well known problem. Therefore, making effort is done for minimizing a used amount of the contrast agent, but the large amount of the contrast agent is necessary for a contrast to difficult lesions such as the LMT lesion or the CTO lesion or the placing of the DES which requires detailed confirmation. As the side effect caused by the contrast agent, there are a renal dysfunction, a dermatopathy, a cardiovascular disability, a respiratory disability and an organa disability etc. Among these side effects, the renal function impairment (contrast-induced nephropathy) becomes particularly problematic since the diabetic patients who receive the PCI have recently increased. When the contrast agent is used for the patients with depressioning of renal function such as renal failure, the contrast agent is not readily eliminated from bodies of the patients so that using such the contrast agent is particularly serious to the patients.

To solve the above-mentioned problems, a prophylactic hemodialysis for removing the contrast agent from a body is carried out after a termination of the PCI and the preoperative examinations. However, during the time at which the contrast agent is injected and the prophylactic hemodialysis is carries out, blood including the contrast agent is not excreted and circulates into an interior of the body so that an overstress to a renes is not prevented. In Coronary Intervention, vol. 2, No. 4 2003, it is described that after the contrast agent is used for patients with chronic kidney disease, dividing the patients into groups; a group (a dialysis group) in which the prophylactic hemodialysis is carried out and a group (non-dialysis group) in which the prophylactic hemodialysis is not carried out to compare an incidence of the contrast-induced nephropathy. As a result, it is reported that there is no difference to be found between the both groups.

Then, an attempt to remove the contrast agent from a body is made not after the PCI and the preoperative examinations is terminated but during the time at which the PCI and the preoperative examinations are carried out. For example, an attempt is made that a catheter is placed into a coronary sinus to let blood including the contrast agent from a body and then the contrast agent is removed through a filter so that the blood is returned to a patient.

In International Publication Nos. WO 02/060511 and WO 02/058777, it is proposed that a catheter having a balloon tip is inserted into a coronary sinus and the balloon tip is temporarily inflated accompanying the injection of the contrast agent while blood flow from the coronary sinus to a right atrium of heart is blocked and blood including the contrast agent is drawn so that the contrast agent is removed by means of CWHD (Continuous Veno-Venous Hemofiltration) or a centrifugation. However, there is a risk of damaging the coronary sinus by expanding the balloon tip as a paries of the coronary sinus is very thin. Moreover, although temporarily, there is a risk that a heart becomes the state of an ischemia by blocking coronary blood. Furthermore, blood plasma component is disposed at the same time when removing the contrast agent. Therefore, in International Publication No. WO 02/060511, a rate of fresh frozen plasma equivalent with the rate of disposed plasma component or albumin is supplemented. However, the supplement of the blood plasma component causes a fear of a potential infection. Therefore, developing a measure for safely removing the contrast agent and relieving the overstress to the renes by the contrast agent is desired.

DISCLOSURE OF INVENTION

To solve the above mentioned problems, the present inventor has invented a contrast agent-removal system and a method of activating the contrast agent-removal system as a result of intently investigation.

Embodiment 1 of the present invention is a contrast agent-removal system comprises a blood suction catheter which can be placed into the coronary sinus, a column for removing a contrast agent from the drawn blood, a blood drawing circuit for directing the blood into the column, a blood pump for controlling the blood drawing amount, a blood return circuit for returning blood from which the contrast agent is removed to a patient's body and a device for measuring a blood suction pressure.

The column for removing a contrast agent from the drawn blood is an adsorber in which an adsorbent for absorbing and removing the contrast agent is filled.

Moreover, as the blood pump for controlling the blood drawing amount is interlocked with the device for measuring a blood suction pressure, the amount of the drawing blood can be controlled by the blood pump according to the blood suction pressure so that blood including the contrast agent can be directed into the adsorber with as much amount of the drawing blood as possible.

Furthermore, when a specific surface area of the adsorbent filled in the adsorber for adsorbing and removing the contrast agent is at least 800 $m^2/g$, a large amount of the contrast agent can be adsorbed.

As the specific surface area of the adsorbent filled in the adsorber for adsorbing and removing the contrast agent is large, an activated charcoal is preferable.

As openings penetrating through an inside of a blood suction catheter are provided on an outer surface of its distal end placed into the coronary sinus, blood including the contrast agent flowing from inlets of coronary veins innumerably concentrating in the coronary sinus can be little biased drawn.

Embodiment 2 of the present invention is the catheter having a blood intake lumen and a blood return lumen together which can be placed into the coronary sinus, the column for removing the contrast agent from the drawn blood, the blood drawing circuit for directing the blood into the column, the blood pump for controlling the blood drawing amount, the blood return circuit for returning the blood from which the contrast agent is removed to a patient's body and the device of measuring a blood suction pressure.

The column of removing the contrast agent from the blood is preferably the adsorber in which the adsorbent for adsorbing and removing the contrast agent is filled.

When the specific surface area of the adsorbent filled in the adsorber for adsorbing and removing the contrast agent is at least 800 $m^2/g$, the large amount of the contrast agent can be adsorbed.

As the specific surface area of the adsorbent filled in the adsorber for adsorbing and removing the contrast agent is large, the activated charcoal is preferable.

According to the contrast agent-removal system of the present invention, it is not necessary that the fresh frozen plasma which is a cause of an infection is supplemented as a loss of the blood plasma component is little. The Embodiment 2 of the present invention is a method of activating the contrast agent-removal system in which the blood is drawn with the blood suction catheter placed into the coronary sinus and the drawn blood is contacted with a contrast agent-adsorbent.

Embodiment 3 of the present invention is the method of activating the contrast agent-removal system in which a flow rate of the blood drawn with the blood suction catheter placed into the coronary sinus is per minute at least 20 mL and at most 200 mL.

The Embodiment 3 is the method of activating the contrast agent-removal system comprising the blood suction catheter, the column in which a contrast agent-adsorbent is filled, the blood drawing circuit for directing the blood into the column, the blood pump for controlling the blood drawing amount, the blood return circuit for returning the blood from which the contrast agent is removed to a patient's body and the device of measuring a blood suction pressure.

BRIEF DESCRIPTION OF DRAWINGS

Fig. 1 is a schematic diagram of one of the embodiments showing a contrast agent-removal system according to Embodiment 1 of the present invention.

Fig. 2 is a schematic diagram showing a distal end placed into the coronary sinus of a blood suction catheter according to Embodiment 1.

Fig. 3 is a schematic sectional view showing an adsorber according to Embodiment 1.

Fig. 4 is a schematic diagram showing the contrast agent-removal system according to Embodiment 2.

Fig. 5 is an explanatory view showing an outer tube and an inner tube which are provided in a coaxial double tubulous shape.

Fig. 6 is an explanatory view showing a biaxial type constitution in which a first lumen and a second lumen are provided in parallel.

Fig. 7 is a view showing a time-lapsed variation of a blood suction pressure in Embodiments.

Fig. 8 is a view showing a time-lapsed variation of a concentration of the contrast agent in blood sampled from an inlet and an outlet of the adsorber in Embodiments and femoral artery.

Fig. 9 is a view showing a time-lapsed variation of the concentration of the contrast agent in blood sampled from the inlet of the adsorber in Embodiments.

Fig. 10 is a view showing a time-lapsed variation of the concentration of the contrast agent in blood sampled from the inlet of the adsorber in Embodiments.

Fig. 11 is a view showing a time-lapsed variation of the concentration of the contrast agent in blood sampled from the inlet of the adsorber in Embodiments.

BEST MODE FOR CARRYING OUT THE INVENTION

EMBODIMENT 1

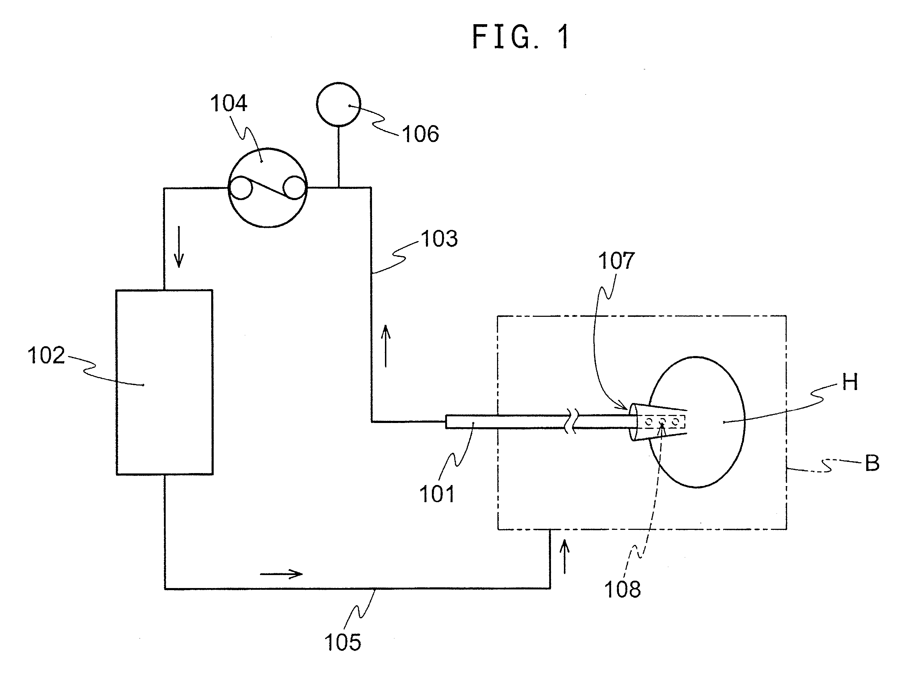

A contrast agent-removal system according to the present Embodiment is shown in Fig. 1. The contrast agent-removal system shown in Fig. 1 includes a blood suction catheter 101 which can be placed into the coronary sinus, a column 102 for removing a contrast agent from the drawn blood, a blood drawing circuit 103 for directing the blood into the column, a blood pump 104 for controlling the blood drawing amount, a blood return circuit 105 for returning blood from which the contrast agent is removed to a patient's body B and a device 106 for measuring a blood suction pressure.

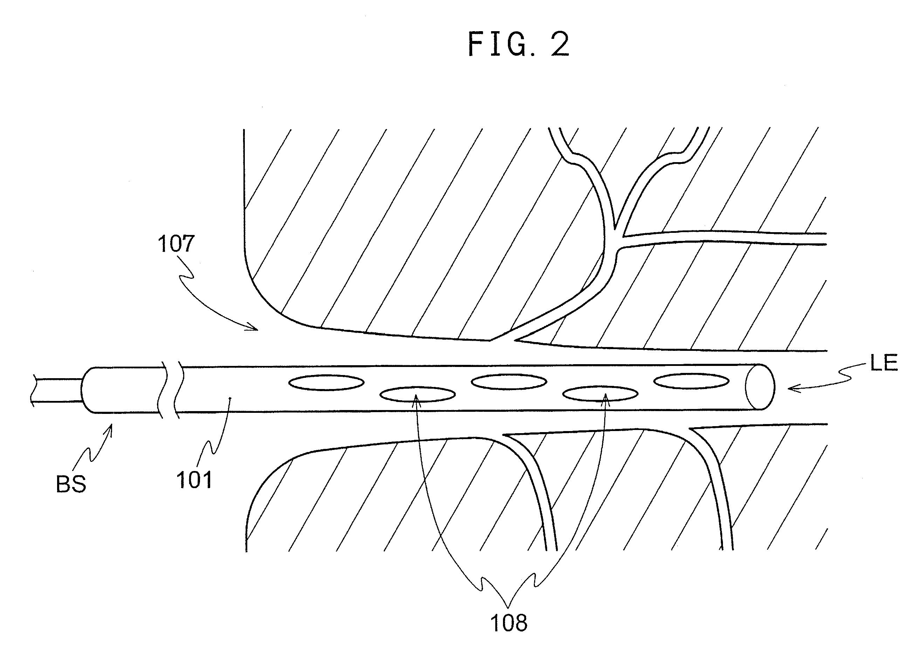

The blood suction catheter 101 is inserted from a patient's femoral vein or cervical vein and can be placed into a coronary sinus 107 of a heart H (see Figs. 1 and 2). Fig. 2 is an enlarged view showing the distal end placed into the coronary sinus of the blood suction catheter 101 according to the Embodiments of the present invention. Referring to Fig. 2, although a structure of the blood suction catheter 101 may be mere a tubulous structure having a lumen from a proximal end to a distal end LE placed into the coronary sinus 107, apertures 108 pierced through the lumen of the catheter are preferably provided on an outer surface of the distal end LE from a viewpoint of reserving a blood drawing amount. The number of the apertures 108 is not limited, but a plurality of the apertures is preferable since innumerable inlets of coronary veins into which the blood containing the contrast agent flowed from a coronary artery flows are concentrated in the coronary sinus 107. A sum (S1) of an area of the apertures 108 is preferably greater than a cross sectional area (SO) of the lumens of the blood suction catheter 101 and S1 is more preferably not less than twice as large as S0. When S1 is greater than thirty times of S0, strength of the catheter decreases. Therefore, S1 is preferably equal to or greater than twice and less than thirty times as large as S0. The apertures 108 may be provided lineally, spirally or in any form in longitudinal axial direction of the blood suction catheter 101. The apertures are completely randomly also provided.

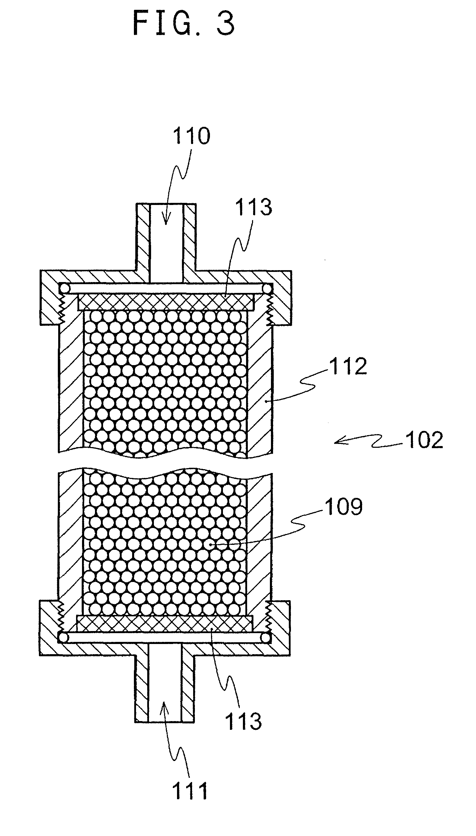

The blood containing the contrast agent drawn with the blood suction catheter 101 is directed into the column 102 through the blood drawing circuit 103. A schematic sectional view of the column 102 of the present Embodiment is shown in Fig. 3. In Fig. 3, 109 is an adsorbent, 110 is an inlet into which blood flows, 111 is an outlet from which blood flows, 112 is a housing and 113 is a mesh. However, the column 102 of the present invention is not limited to such specific example. For example, a container including an inlet and an outlet for blood, and a means for preventing the adsorbent from flowing out of the container in which the adsorbent adsorbing the contrast agent is filled can be used and the shape thereof is not particularly limited.

As shown in Fig. 3, the adsorbent 109 is filled in the column 102. The adsorbent 109 is a water-insoluble adsorbent which physiochemically adsorbs the contrast agent. The adsorbent in the present invention is preferably porous from a viewpoint that a specific surface area of the contrast agent can be reliably provided. The porous body includes numerous and continuous pores or discontinuous pores. The specific surface area of the adsorbent is preferably not less than 800 $m^2/g$, since an amount of adsorbing the contrast agent is large. As the porous body, there is such as silica gel, alumina gel, zeolite and activated charcoal, though an activated charcoal is most preferable from a viewpoint of a large specific surface area.

As a shape of the adsorbent, any of spherical shape, granulated shape, filate shape, hollow fiber shape and flat membrane shape is effectively used. Among these, spherical shape or granulated shape is most preferably used from a viewpoint of blood circulation in extracorporeal circulation.

Though the adsorbent contacts with blood, the blood coagulates when the blood contacts with a foreign particle. Therefore, the adsorbent of the present invention may be subjected to a surface treatment for controlling an adhesion of a blood platelet to improve an affinity with blood. For example, a coat layer which is a material with a low adhesion of the blood platelet is preferably provided on the surface of the adsorbent with which the blood contacts.

The blood from which the contrast agent is removed in the column 102 is returned to a peripheral blood vessel such as a femoral vein of a patient through the blood return circuit 105.

The blood pump 104 of the present Embodiment is a pump for directing the blood containing the contrast agent which is drawn with the blood suction catheter 101 into the column 102 through the blood drawing circuit 103. Furthermore, the device 106 for measuring the blood suction pressure measures the blood suction pressure which is measured between the blood suction catheter 101 and the blood pump 104. The blood suction pressure of the present invention is represented by a deference of both pressures (P1 - PO); a pressure measured by the device 106 for measuring is P0 mmHg when the blood flow rates are 0 mL/min and a pressure measured by the device 106 for measuring is P1 mmHg when the blood flow rates are Q1 mL/min (Q1 > 0) . As the amount of the drawn blood increases, a value of P1 decreases, so the blood suction pressure becomes a negative pressure. When the blood suction pressure extremely becomes the negative pressure, flattened blood vessel or significant occlusion of the blood vessel is caused, so an overstress is given to a patient. When the blood suction pressure becomes less than -200 mmHg, there is an extreme high probability that the blood vessel is occlusive. Therefore, the blood suction pressure is preferably equal to or greater than -200 mmHg. The blood suction pressure is more preferably equal to or greater than -150 mmHg to prevent the flattened blood vessel. The blood suction pressure is furthermore preferably equal to greater than -100 mmHg. To remove efficiently the contrast agent, the amount of the drawn blood directed into the column 102 may be as large as possible. In the present invention, the amount of the drawn blood can be controlled by the blood pump 104 in accordance with the blood suction pressure while the blood suction pressure is monitored by the device 106 for measuring the blood suction pressure, and this control may be carried out manually or automatically. The blood containing the contrast agent can be directed into the column 102 with much the amount of the drawn blood as possible while the blood suction pressure is controlled.

A contrast agent aimed at by the contrast agent-removal system of the present Embodiment is discriminated by x-ray and a compound including ionic or non-ionic iodine. A molecular weight of the contrast agent used for being administered into blood vessel is approximately less than 8000. As a concrete example, there are a monomer such as iopromide, iopamidol, iomeprol, amidotrizoic acid, iohexol, iothalamic acid, iodamide, metrizoic acid, metrizamide and ioxilan, and a dimmer such as ioxaglic acid, adipiodone, iotroxic acid, iodoxamic acid, iotrolan. The compound is not limited to these.

EMBODIMENT 2

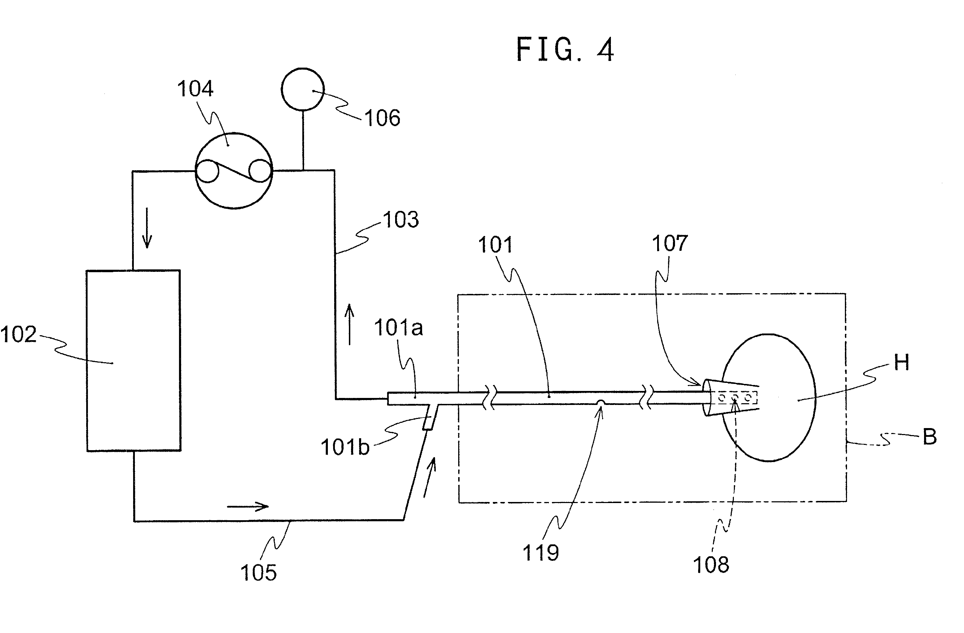

A schematic diagram of the contrast agent-removal system of the present Embodiment is shown in Fig. 4. The contrast agent-removal system includes the blood suction catheter 101 which can be placed into the coronary sinus 107 of the heart H (see Figs. 1 and 4), the column 102 for removing the contrast agent in the drawn blood, the blood drawing circuit 103 for directing the blood into the column 102, the blood pump 104 for controlling the amount of the drawn blood, the blood return circuit 105 for returning blood from which the contrast agent is removed to a patient's body B and the device 106 for measuring the blood suction pressure.

Referring to Fig. 4, the blood suction catheter 101 of the present Embodiment includes a main tube 101a including a blood intake lumen 115 and a blood return lumen 116 and a branch tube 101b which is connected to the tube 101a and in communication with the blood intake lumen 115. The blood suction catheter 101 is composed of the blood intake lumen 115 and the blood return lumen 116 together.

The contrast agent-removal system of the present Embodiment is only different in a structure of the catheter as compared to the contrast agent-removal system of the Embodiment 1. Other constituent element which composes the contrast agent-removal system is the same as the constituent element of the Embodiment 1. Therefore an explanation of other constituent element is abbreviated. In terms of the contrast agent employed by the contrast agent-removal system of the present Embodiment, it goes without saying that the contrast agents exemplified in the Embodiments 1 are applied.

The structure of the blood suction catheter 101 of the present Embodiment is explained in the following.

The blood suction catheter 101 of the present Embodiment is inserted from a patient's femoral vein or cervical vein and can be placed into the coronary sinus 107. The blood suction catheter 101 also includes the blood intake lumen 115 and the blood return lumen 116.

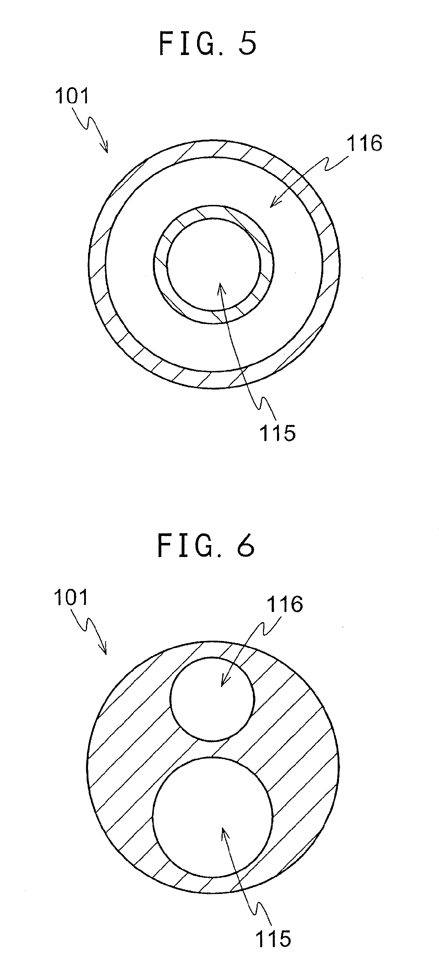

In the present Embodiments, when the blood suction catheter 101 includes at least the first and second lumens, the lumen 115 and the lumen 116, a structure of the blood suction catheter 101 is not limited. In other words, as shown in Fig. 5, the co-axial type structure with the inner tube and the outer tube provided in a coaxial double tubulous shape may be used in which a first lumen 115 defined by an inner face of the inner tube and a second lumen 116 defined by an inner face of the outer tube and outer face of the inner tube are included. As shown in Fig. 6, the bi-axial type structure may be used in which the first lumen 115 and the second lumen 116 are provided in parallel.

In the present Embodiment, the blood from which the contrast agent is removed in the column 102 is directed into the blood suction catheter 101 through the blood return circuit 105 and then the blood is returned to a patient's peripheral blood vessel such as femoral vein from an aperture 119 pierced through the catheter 101 (see Fig. 4).

The catheter of the present Embodiment includes the blood intake lumen and a blood return lumen together. Therefore, only one site for inserting and placing the catheter may be needed, and there is no need to provide another blood access site. Therefore, only one site needs to be needled, in addition a possibility of leakage of the blood from the needled site can be reduced and an invasion to the patient can also be reduced. Moreover, a burden to an operator can be reduced since an excessive extension of the operation time is not accompanied.

EXAMPLE

The present invention will be explained in detail with following examples, but the present invention is not limited to the following examples.

EXAMPLE 1

To LWD pig having a weight of 54.9 kg, the blood suction catheter was inserted from a sheath fixed to a right femoral vein of the pig, and then the distal end of the blood suction catheter having apertures was placed into the coronary sinus of the pig under an inhalation anesthesia. The one end of the blood drawing circuit was connected to the proximal end of the blood suction catheter for extracting blood and the other end of the blood drawing circuit was connected to the inlet of the adsorber shown in Fig. 3 into which blood flows by way of the blood pump. In the present Example, activated charcoal was used as the adsorber. Subsequently, the one end of the blood return circuit was connected to the outlet of the adsorber from which blood flows and the other end of the blood return circuit was connected to a sheath fixed to a right cervical vein of the piglet. Moreover, the device for measuring the blood suction pressure was connected to the blood drawing circuit between the blood suction catheter and the blood pump. While observing the blood suction pressure, the blood pump was started to circulate blood. When the blood flow rates were up to 30 mL/min, the blood suction pressure was approximately -30 mmHg. Moreover, the blood flow rates increased to 35 mL/min, and then the blood suction pressure rapidly decreases nearly to -100 mmHg. Therefore, a blood circulation was carried out with 30 mL/min of the blood flow rates.

A guiding catheter was inserted into a sheath fixed to a left cervical vein of the piglet, and then placed into a left coronary artery of the piglet with 15 mL/min of the contrast agent continuously being injected. As a result, 150 mL of the contrast agent in total was injected into the coronary artery of the piglet.

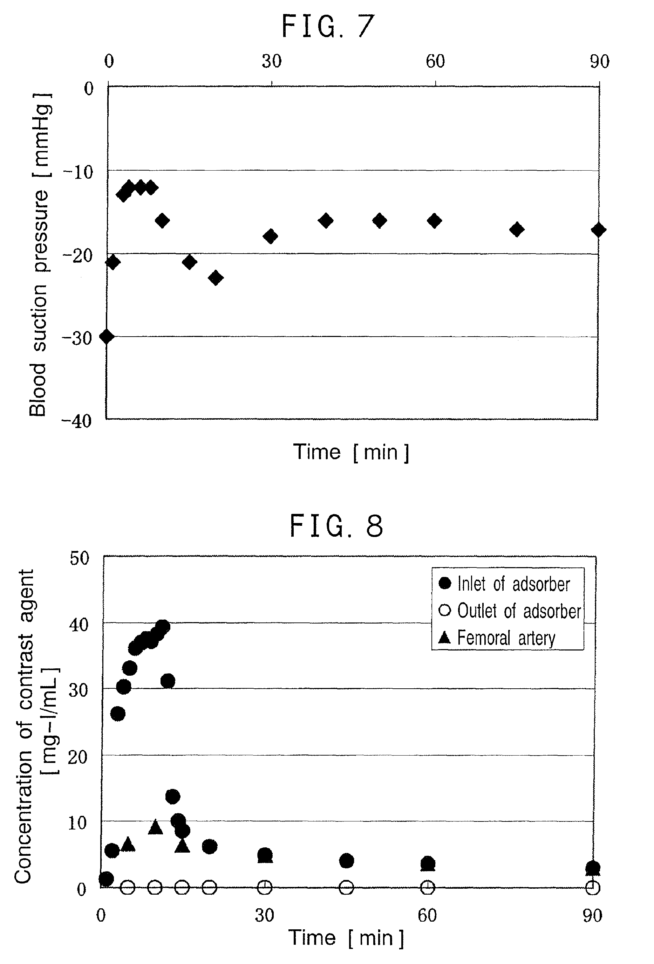

The blood suction pressure at a time when the blood circulation was carried out with 30 mL/min of the blood flow rates for 90 minutes from an initiation of injecting the contrast agent was measured. The result is shown in Table 1 and Fig. 7.

TABLE 1

| Time (min) | Blood suction pressure (mmHg) |
|---|---|
| 0 | -30 |
| 1 | -21 |
| 3 | -13 |
| 4 | -12 |
| 6 | -12 |
| 8 | -12 |
| 10 | -16 |
| 15 | -21 |
| 20 | -23 |
| 30 | -18 |
| 40 | -16 |
| 50 | -16 |
| 60 | -16 |
| 75 | -17 |
| 90 | -17 |

The blood was sampled from the inlet into which blood flows, the outlet from which blood flows of the adsorber and a femoral artery of the piglet over time. The collected blood was subjected to a centrifugation to separate the blood plasma. Then an absorbance in a wavelength with a range from nearly 240 to 250 nm was measured by a spectrophotometer and the measured absorbance was compared with that of a contrast agent with standard concentration, so the concentration of the contrast agent in the blood was measured. The result is shown in Table 2 and Fig. 8.

TABLE 2

| Time (min) | Concentration of contrast agent (mg-I/mL) | | |
|---|---|---|---|
| | Inlet of adsorber | Outlet of adsorber | Femoral artery |
| 1 | 1.3 | | |
| 2 | 5.5 | | |
| 3 | 26.1 | | |
| 4 | 30.3 | | |
| 5 | 33.1 | 0.0 | 6.6 |
| 6 | 36.0 | | |
| 7 | 36.9 | | |
| 8 | 37.5 | | |
| 9 | 37.2 | | |
| 10 | 38.1 | 0.0 | 9.2 |
| 11 | 39.2 | | |
| 12 | 31.1 | | |
| 13 | 13.7 | | |
| 14 | 10.1 | | |
| 15 | 8.6 | 0.0 | 6.4 |
| 20 | 6.2 | 0.0 | |
| 30 | 4.9 | 0.0 | 4.8 |
| 45 | 4.0 | 0.0 | |
| 60 | 3.5 | 0.0 | 3.7 |
| 90 | 3.1 | 0.0 | 3.1 |

It can be understood from the result of Table 1 and Fig. 7 that the blood circulation was stably carried out for 90 minutes with the blood suction pressure being remained in nearly -20 mmHg.

It can be understood form the result of Table 2 and Fig. 8 that the contrast agent injected from the coronary artery of the piglet was efficiently drawn from the coronary sinus of the piglet and a circulation of the contrast agent through a whole body was prevented since the concentration of the contrast agent in the femoral artery was suppressed to a low concentration as compared to that of the contrast agent in the inlet of the adsorber. Furthermore, all of the contrast agent flowing into the adsorber could be adsorbed and removed since the concentration of the contrast agent in the outlet of the adsorber was zero.

In the present Example, only a fluid infusion (60 mL/h of lactate Ringer solution, 352 mL of the fluid infusion in total) was carried out.

EXAMPLE 2

To LWD pig having a weight of 49.3 kg, the blood suction catheter was inserted from the right femoral vein of the pig, and then the distal end of the catheter having the apertures was placed into the coronary sinus of the pig under the inhalation anesthesia. The one end of the blood drawing circuit was connected to the proximal end of the blood suction catheter and the other end of the blood drawing circuit was connected to the inlet of the adsorber (580 mL of activated charcoal coated with poly-HEMA was filled) into which blood flows shown in Fig. 3 by way of the blood pump. Subsequently, the one end of the blood return circuit was connected to the outlet of the adsorber from which blood flow and the other end of the blood return circuit was connected to a left femoral vein of the piglet. Moreover, the device for measuring the blood suction pressure was connected to the blood drawing circuit between the blood suction catheter and the blood pump. A balloon catheter for vasodilation was inserted from the left cervical vein of the pig and the balloon catheter was placed into an azygos vein (blood vessel peculiar to pig and human beings don't have such as blood vessel) and inflated. The balloon catheter was inserted from the right cervical vein and then the catheter was placed into the coronary sinus and then the balloon for vasodilation was inflated, so blood flow from the coronary sinus to a right atrium of heart was blocked. From the guiding catheter which was inserted from the left cervical vein and placed into the left coronary artery, 2.4 mL/min (corresponding to 888 mg-I/min) of the contrast agent was continuously injected into the coronary artery for 60 minutes while an extracorporeal circulation is carried out by 60 mL/min of the blood flow rates for 90 minutes.

Then, after the balloon for vasodilation was deflated and the blocking of the blood flow was released, the contrast agent was administered in the same manner as described above while an extracorporeal circulation was carried out.

A time-lapsed variation of the concentration of the contrast agent in blood sampled from the inlet of the adsorber was compared in both cases; a case in which the balloon for vasodilation was inflated and a case in which the balloon for vasodilation is not inflated. With respect to a case in which the balloon for vasodilation was not inflated, the concentration of the contrast agent in blood sampled from the outlet of the adsorber was measured in the same manner as Embodiment 1. In addition, an interval adsorbed amount in every minute when blood is drawn was calculated by the following formula 1 from the concentration of the contrast agent in the inlet and the outlet of the adsorber and the amount of treated blood.

Blood sampling time 1 (min): t1
Blood sampling time 2 (min): t2
Concentration of a contrast agent on the side of an inlet in a blood sampling time 1 (mg-I/mL): Ci1
Concentration of a contrast agent on the side of an inlet in a blood sampling time 2 (mg-I/mL): Ci2
Concentration of a contrast agent on the side of an outlet in a blood sampling time 1 (mg-I/mL): Co1
Concentration of a contrast agent on the side of an outlet in a blood sampling time 2 (mg-I/mL): Co2
Hematocrit in sampled blood: Hct
Blood flow rates: F
Wherein t2 > t1

$$(Ci1 - Co1 + Ci2 - Co2) \times F \times Hct \times (t2 - t1)/2 \qquad (Formula\ 1)$$

Wherein t2 > t1
In addition, Hct was 0.68.

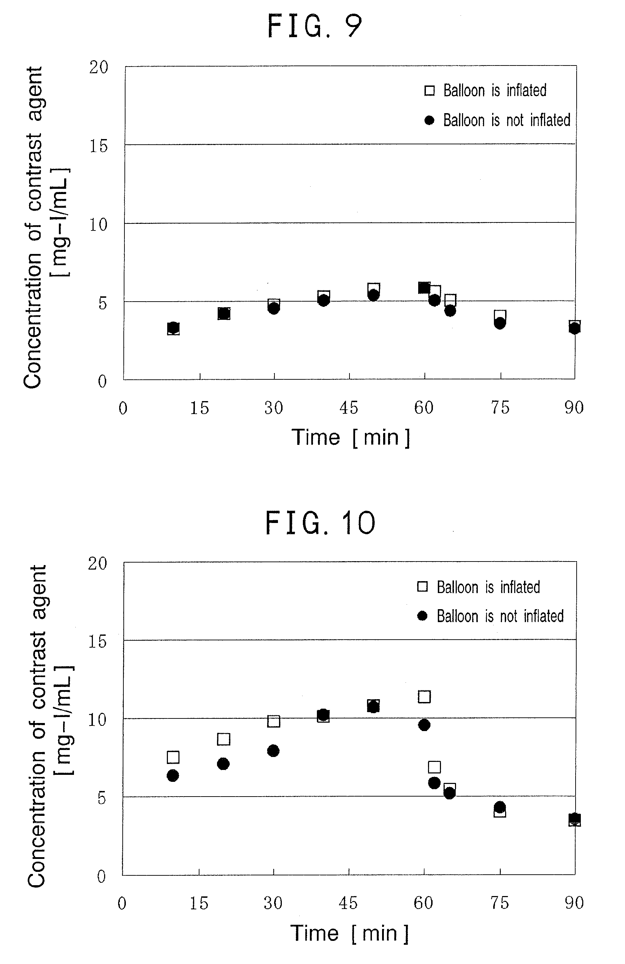

The result is shown in Table 3 and Fig. 9.

TABLE 3

| Time (min) | Concentration of contrast agent in inlet of adsorber (mg-I/mL) | | Concentration of contrast agent in outlet of adsorber (mg-I/mL) | Interval adsorbed amount (mg-I) |
|---|---|---|---|---|
| | Balloon is inflated | Balloon is not inflated | Balloon is not inflated | Balloon is not inflated |
| 0 | 1.3 | 1.4 | 0.0 | - |
| 10 | 3.2 | 3.3 | 0.0 | 961 |
| 20 | 4.2 | 4.2 | 0.0 | 1524 |
| 30 | 4.7 | 4.5 | 0.0 | 1772 |
| 40 | 5.3 | 5.0 | 0.0 | 1947 |
| 50 | 5.7 | 5.3 | 0.0 | 2111 |
| 60 | 5.9 | 5.8 | 0.0 | 2266 |
| 62 | 5.6 | 5.0 | 0.0 | 440 |
| 65 | 5.0 | 4.3 | 0.0 | 571 |
| 75 | 4.0 | 3.5 | 0.0 | 1598 |
| 90 | 3.3 | 3.2 | 0.0 | 2050 |

It is understood from the result of Table 3 and Fig. 9 that the concentration of the contrast agent in the inlet of the adsorber is equivalent in the both cases, and blood including the contrast agent with high concentration can be directed into the column even when the balloon is not inflated in the same extent as when the balloon is inflated. It is furthermore understood from a sum of the interval adsorbed amount that 15293 mg-I (corresponding to 41 mL) of the contrast agent can be adsorbed and removed when the balloon is not inflated.

EXAMPLE 3

The extracorporeal circulation was performed in the same manner as Embodiment 2 except that the administered amount of the contrast agent was 3.1 mL/min (corresponding to 1147 mg-I/min) and the blood flow rates in the extracorporeal circulation were 70 mL/min. Then, a time-lapsed variation of the concentration of the contrast agent in blood was compared. In addition, Hct was 0.67. The result is shown in Table 4 and Fig. 10.

TABLE 4

| Time (min) | Concentration of contrast agent in inlet of adsorber (mg-I/mL) | | Concentration of contrast agent in outlet of adsorber (mg-I/mL) | Interval adsorbed amount (mg-I) |
|---|---|---|---|---|
| | Balloon is inflated | Balloon is not inflated | Balloon is not inflated | Balloon is not inflated |
| 0 | 0.6 | 1.7 | 0.0 | - |
| 10 | 7.4 | 6.3 | 0.0 | 1869 |
| 20 | 8.6 | 7.0 | 0.0 | 3127 |
| 30 | 9.8 | 7.8 | 0.1 | 3459 |
| 40 | 10.1 | 10.2 | 0.7 | 4032 |
| 50 | 10.8 | 10.7 | 3.0 | 4026 |
| 60 | 11.3 | 9.5 | 5.2 | 2819 |
| 62 | 6.8 | 5.8 | 5.7 | 207 |
| 65 | 5.4 | 5.1 | 5.0 | 14 |
| 75 | 4.0 | 4.2 | 2.5 | 437 |
| 90 | 3.5 | 3.5 | 2.0 | 1158 |

It is understood from the result of Table 4 and Fig. 10 that the concentration of the contrast agent in the inlet of the adsorber is equivalent between the both cases, and blood including the contrast agent with high concentration can be directed into the column even when the balloon is not inflated in the same extent as when the balloon is inflated. It is furthermore understood from the sum of the interval adsorbed amount that 21149 mg-I (corresponding to 57 mL) of the contrast agent can be adsorbed and removed when the balloon is not inflated.

EXAMPLE 4

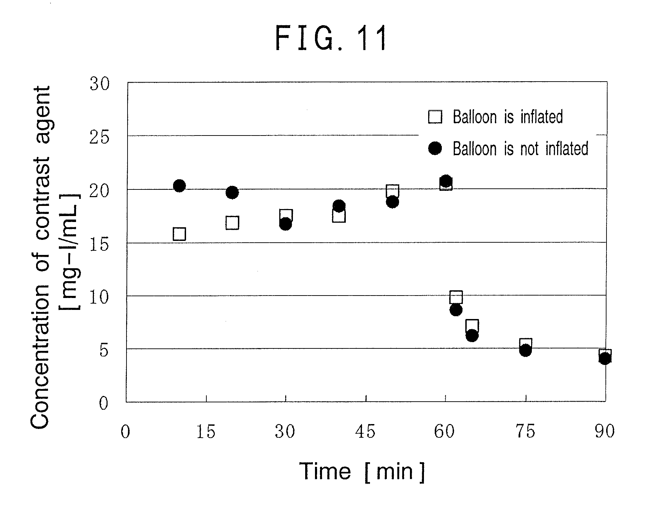

The extracorporeal circulation was performed in the same manner as Example 2 except that the administered amount of the contrast agent was 2.6 mL/min (corresponding to 962 mg-I/min) and the blood flow rates in the extracorporeal circulation were 70 mL/min. Then, a time-lapsed variation of the concentration of the contrast agent in blood is compared. In addition, Hct was 0.62. The result is shown in Table 5 and Fig. 11.

TABLE 5

| Time (min) | Concentration of contrast agent in inlet of adsorber (mg-I/mL) | | Concentration of contrast agent in outlet of adsorber (mg-I/mL) | Interval adsorbed amount (mg-I) |
|---|---|---|---|---|
| | Balloon is inflated | Balloon is not inflated | Balloon is not inflated | Balloon is not inflated |
| 0 | 0.7 | 2.5 | 0.0 | - |
| 10 | 15.7 | 20.3 | 0.0 | 4937 |
| 20 | 16.8 | 19.7 | 0.0 | 8662 |
| 30 | 17.4 | 16.6 | 2.4 | 7362 |

(continued)

| Time (min) | Concentration of contrast agent in inlet of adsorber (mg-I/mL) | | Concentration of contrast agent in outlet of adsorber (mg-I/mL) | Interval adsorbed amount (mg-I) |
|---|---|---|---|---|
| | Balloon is inflated | Balloon is not inflated | Balloon is not inflated | Balloon is not inflated |
| 40 | 17.4 | 18.3 | 7.8 | 5374 |
| 50 | 19.7 | 18.7 | 11.5 | 3861 |
| 60 | 20.4 | 20.6 | 13.9 | 3036 |
| 62 | 9.8 | 8.6 | 14.1 | 53 |
| 65 | 7.0 | 6.1 | 14.2 | 0 |
| 75 | 5.2 | 4.7 | 4.1 | 0 |
| 90 | 4.3 | 3.9 | 2.8 | 586 |

It is understood from the result of Table 5 and Fig. 11 that the concentration of the contrast agent in the inlet of the adsorber is equivalent between the both cases, and blood including the contrast agent with high concentration can be directed into the column even when the balloon is not inflated in the same extent as when the balloon is inflated. It is furthermore understood from the sum of the interval adsorbed amount that 33871 mg-I (corresponding to 92 mL) of the contrast agent can be adsorbed and removed when the balloon is not inflated.

INDUSTRIAL APPLICABILITY

According to the contrast agent-removal system of the present invention, removing efficiently the contrast agent which is the cause of the renal function impairment is possible during the PCI and the preoperative examinations.

The contrast agent-removal system of the present invention can direct the blood containing relatively high concentration of contrast agent into a column by drawing blood from a coronary sinus. In the contrast agent-removal system, the higher the concentration of the contrast agent in the blood flowing into the column is, the higher removing performance can be exhibited, the higher ratio of removing the contrast agent can be obtained. Therefore, removing efficiently the contrast agent which is the cause of the renal function impairment is possible during the PCI and the preoperative examinations.

In the contrast agent-removing system of the present invention, the amount of the drawn blood can be controlled in accordance with the blood suction pressure while the blood suction pressure is monitored. Therefore, the blood can be directed into the column with much the amount of the drawn blood as possible.

In the contrast agent-removal system of the present invention, as openings penetrated through an inside of the blood intake lumen are provided on an outer surface of a distal end placed into the coronary sinus, the blood containing the contrast agent flowing from inlets of coronary veins which innumerably concentrate in the coronary sinus can be evenly drawn.

Furthermore, the possibility is decreased that the distal end of the catheter and a wall of the coronary sinus adhere to each other, so the amount of the drawn blood is secured. Furthermore, the possibility is decreased that periphery wall of the coronary sinus is damaged or perforating is caused.

According to the contrast agent-removal system of the present invention, as a catheter balloon for drawing blood such as a conventional one is not needed, there is no risk of damaging the coronary sinus by inflating of the balloon.

According to the contrast agent-removal system of the present invention, as loss of blood plasma component is reduced, there is no need for supplementing such as fresh frozen plasma which causes infection.

In addition, the large amount of the contrast agents can be adsorbed by using the adsorbent for adsorbing the contrast agent with equal to or greater than 800 m$^2$/g of specific surface area.

Furthermore, when the adsorbent for adsorbing the contrast agent including activated charcoal as a component is used, the specific surface area of the adsorbent for adsorbing the contrast agent is sufficiently large. Therefore, the large amount of the contrast agents can be adsorbed.

According to Embodiment 2 in the present invention, by using a catheter which includes the blood intake lumen and a blood return lumen together and which can be placed into the coronary sinus, only one site for inserting and placing the catheter may be needed and there is no need to provide another blood access site for returning blood. Therefore, only one site needs to be needled, in addition a possibility of leakage of the blood from the needled site can be reduced and an invasion to the patient can also be reduced. Moreover, a burden to an operator can be reduced since an excessive extension of the operation time is not accompanied.

According to Embodiment 3 of the present invention, removing efficiently the contrast agent from blood containing the contra agent is possible.

According to Embodiment 3 of the present invention, it is characterized that the blood suction pressure is controlled equal to or greater than -200 mmHg. Ischemic state of a heart is avoided and the large amount of the contrast agents can be adsorbed.

Moreover, the contrast agent-removal system is operated with the use of the blood suction catheter with the openings penetrated through an outer surface of a distal end placed into the coronary sinus to an inside of the blood suction catheter, so the blood containing the contrast agent flowing from inlets of the coronary veins which innumerably concentrate in the coronary sinus can be little biased drawn.

**Claims**

1. A contrast agent-removal system comprising:

   a blood suction catheter which can be placed into the coronary sinus;
   a column for removing a contrast agent from the drawn blood;
   a blood drawing circuit for directing the blood into the column;
   a blood pump for controlling an amount of the drawn blood;
   a blood return circuit for returning the blood from which the contrast agent is removed to a patient's body; and
   a device for measuring a blood suction pressure.

2. A contrast agent-removal system comprising:

   a catheter having a blood intake lumen and a blood return lumen together, and being able to be placed into the coronary sinus;
   a column for removing a contrast agent from the drawn blood;
   a blood drawing circuit for directing the blood into the column;
   a blood pump for controlling an amount of the drawn blood;
   a blood return circuit for returning the blood from which the contrast agent is removed to the patient's body; and
   a device for measuring a blood suction pressure.

3. The contrast agent-removal system according to Claim 1 or 2, wherein the blood pump for controlling the amount of the drawn blood operates in conjunction with the device for measuring a blood suction pressure.

4. The contrast agent-removal system according to Claim 1, 2 or 3, wherein openings penetrated through an inside of the blood suction catheter are provided on an outer surface of its distal end placed into the coronary sinus.

5. The contrast agent-removal system according to Claim 1, 2, 3, or 4, wherein the column for removing the contrast agent in the blood is an adsorber in which an adsorbent for adsorbing and removing the contrast agent is filled.

6. The contrast agent-removal system according to Claim 5, wherein a specific surface area of the adsorbent filled in the adsorber for adsorbing and removing the contrast agent is equal to or greater than 800 $m^2/g$.

7. The contrast agent-removal system according to Claim 5 or 6, wherein the adsorbent filled in the adsorber for adsorbing and removing the contrast agent is activated charcoal.

8. A method of operating a contrast agent-removal system comprising steps of:

   drawing blood with a blood suction catheter placed into the coronary sinus; and
   contacting the blood with an adsorbent for adsorbing a contrast agent.

9. A method of operating a contrast agent-removal system, wherein blood flow rates drawn with a blood suction catheter placed into the coronary sinus is equal to or greater than 20 mL/min and equal to or less than 200 mL/min.

10. A method of operating a contrast agent-removal system, wherein blood is drawn with a blood suction catheter placed into the coronary sinus and the blood is contacted with an adsorber for adsorbing a contrast agent in the contrast agent-removal system according to Claim 1 or 2.

11. A method of operating a contrast agent-removal system, wherein a flow rate of blood drawn with a blood suction catheter placed into the coronary sinus is equal to or greater than 20 mL/min and equal to or less than 200 mL/min in the contrast agent-removal system according to Claim 1 or 2.

12. The method of operating the contrast agent-removal system according to Claim 8 or 9, wherein the blood suction catheter with openings is used in which the openings are penetrated through an outer surface of the distal end placed into the coronary sinus to an inside of the catheter.

13. The method of operating the contrast agent-removal system according to Claim 8 or 9, wherein the blood suction pressure is controlled equal to or greater than -200 mmHg.

14. The method of operating the contrast agent-removal system according to Claim 8, wherein the adsorbent for adsorbing the contrast agent whose the specific surface area is equal to or greater than 800 m$^2$/g is used.

15. The method of operating the contrast agent-removal system according to Claim 8, wherein the adsorbent for adsorbing the contrast including activated charcoal as a component is used.

# FIG. 1

EP 1 859 826 A1

# FIG. 2

EP 1 859 826 A1

# FIG. 3

EP 1 859 826 A1

FIG. 4

# FIG. 5

# FIG. 6

## FIG. 7

## FIG. 8

## FIG. 9

## FIG. 10

## FIG. 11

## INTERNATIONAL SEARCH REPORT

| International application No. |
| --- |
| PCT/JP2006/303079 |

A. CLASSIFICATION OF SUBJECT MATTER
***A61M1/36*** (2006.01), *A61K49/04* (2006.01)

According to International Patent Classification (IPC) or to both national classification and IPC

B. FIELDS SEARCHED

Minimum documentation searched (classification system followed by classification symbols)
A61M1/36, A61K49/04

Documentation searched other than minimum documentation to the extent that such documents are included in the fields searched
Jitsuyo Shinan Koho       1922-1996   Jitsuyo Shinan Toroku Koho   1996-2006
Kokai Jitsuyo Shinan Koho  1971-2006   Toroku Jitsuyo Shinan Koho   1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 05-049694 A (Asahi Medical Co., Ltd.), 02 March, 1993 (02.03.93), Par. Nos. [0008], [0016], [0017]; all drawings (Family: none) | 1-7,9,11 |
| Y | JP 2005-503183 A (The Regents of the University of California), 03 February, 2005 (03.02.05), Full text; all drawings & WO 2002/058777 A2  & US 2002/0099254 A1 | 1-7,9,11 |
| Y | JP 04-075663 A (Research Development Corp. of Japan), 10 March, 1992 (10.03.92), Page 2, lower left column, line 17 to lower right column, line 1; all drawings (Family: none) | 3 |

| ☒ Further documents are listed in the continuation of Box C. | ☐ See patent family annex. |
| --- | --- |

\* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 19 April, 2006 (19.04.06) | 02 May, 2006 (02.05.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

**EP 1 859 826 A1**

**INTERNATIONAL SEARCH REPORT**

| International application No. |
| PCT/JP2006/303079 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2003-154008 A  (Terumo Cardiovascular Systems Corp.), 27 May, 2003 (27.05.03), Par. No. [0014]; all drawings & US 2003/0078564 A1    & EP 1304133 A2 | 4 |

Form PCT/ISA/210 (continuation of second sheet) (April 2005)

22

## INTERNATIONAL SEARCH REPORT

| International application No. |
| --- |
| PCT/JP2006/303079 |

**Box No. II        Observations where certain claims were found unsearchable (Continuation of item 2 of first sheet)**

This international search report has not been established in respect of certain claims under Article 17(2)(a) for the following reasons:

1. ☒ Claims Nos.: 8, 10, 12-15
    because they relate to subject matter not required to be searched by this Authority, namely:

    Claims 8, 10, and 12 to 15 pertain to methods for treatment of the human body by therapy and thus relate to a subject matter which this International Searching Authority is not required, under the provisions of the PCT Rule 39.1(iv), to search.

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**
the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02060511 A

- WO 02058777 A

**Non-patent literature cited in the description**

- *Coronary Intervension,* 2003, vol. 2 (4